# EUROPEAN PATENT APPLICATION

(11) **EP 1 398 034 A1**
(43) Date of publication of application: **17.03.2004**
(21) Application number: 03019519.2
(22) Date of filing: 29.08.2003
(51) Int. Cl.: A61K 35/78, A61K 7/00, A61P 17/00, A61P 33/14

(54) **Topical application composition for preventing and treating pediculosis, method of elaboration and uses thereof**

(30) Priority: 13.09.2002 AR 0213480
(71) Applicant: Roldan, Vicente Teofilo, 5500 Mendoza (AR); Barboza, Juan José, 5519 Mendoza (AR)
(72) Inventor: Roldan, Vicente Teofilo, 5500 Mendoza (AR); Barboza, Juan José, 5519 Mendoza (AR)
(74) Representative: Robba, Pierpaolo

(57) **Abstract**

A composition of topical application for the prevention and treatment of pediculosis, elaboration method and uses of said composition. The composition comprises at least between 0.2 and 0.3% (w/v) of diallil tiosulphinate, wherein the diallil tiosulphinate is extracted from the garlic plant*(Allium sativum* sp). The composition may also comprise between 0.06 and 0.1% (w/v) of other tiosulphinates and sulfur compounds deriving therefrom, all of them extracted from the garlic plant *(Allium* sativum sp).

The method comprises the stages of: a) peeling and grinding garlic cloves until a homogeneous paste is obtained; b) heating a quantity of water until boiling and further addition of a quantity of organic acid, for example, citric acid, until an aqueous solution is obtained, of pH between 3 and 3.5; c) immersing a quantity of the garlic paste obtained in stage a) in a quantity of the aqueous boiling solution of stage b); and d) cooling and fractioning the aqueous solution in stage c), wherein said aqueous solution comprises diallil tiosulphinate, other tiosulpinates and other sulfur compounds deriving therefrom.

The composition of the invention is used for the elaboration of a medicine or a cosmetic product for pediculosis, wherein said medicine or cosmetic product is presented under hair lotion, hair cream, hair conditioner, shampoo, hair rinse cream, hair gel and topical application powder formulations.

## Description

The present invention relates to a topical application composition for preventing and treating pediculosis, a method of elaboration and the uses thereof. More particularly, the invention refers to a composition comprising between 0.2 and 0.3 % (w/v) of diallil tiosulphinate (allicin), wherein the diallil tiosulphinate compound is extracted from the garlic plant (*Allium sativum sp*). The composition may also comprise between 0.06 and 0.1 % (w/v) of other tiosulphinates and sulphur compounds deriving therefrom, all of them extracted from the garlic plant (*Allium sativum sp*). The composition of the invention may be in the form of a hair lotion, a hair cream, a hair rinse, a shampoo, a hair gel or a topical application powder and in any other cosmetic form included in Resolution 155/98, National Ministry of Public Health.

### BACKGROUND OF THE INVENTION

Lice are arthropods belonging to the order *Phthiraptera* (wingless insects), specifically to the suborder *Anoplura* (sucking lice) and to the *Pediculidae* and *Phthiriidae* families. *Pediculus humanus* and *Phthirus pubis* (pubic lice) are the two species that infest human beings. *P.humanus* divides into two subspecies: *Pediculus humanus capitis* (head louse) and *Pediculus humanus corporis* (body louse). Infestation with any of these parasites is called pediculosis.

Infestation is spread among people by direct contact or by sharing personal articles such as combs and brushes. During the development of the disease there is a first incubation period, during which the person has no symptoms but is able to spread the disease.

Lice lay eggs (nits) on the hair close to the scalp. Eggs are oval in shape and hatch into naiads after a 4-15 day period through incomplete metamorphosis. Before dying, adult female lice lay from 50 to 100 eggs on the hair. The complete cycle takes approximately one month.

It is known that lice have troubled human beings since ancient times and have been found in all cities worldwide. Lice infestation is a serious problem, not only because it causes skin irritation, but also because it transmits diseases such as epidemic typhus, trench fever and so forth.

Lice are host specific. For example, dog lice do not parasitize on human beings and viceversa.

*Pediculosis capitis* treatment requires the thorough removal of eggs and lice.

To date the main treatment is based on Pyrethrines and Lindane. Permethrin, a synthetic derivate from pyrethrines, is considered the recommended therapy. In general, it is commercially available in the form of a hair conditioner or shampoo. Some of the commercially available permethrins are ovicides and have a 7-10 day residual activity. Permethrines must be carefully used in allergic persons, since they may cause breathing disorders.

Treatments with lindane-based products must be carefully applied. The frequent use, repeated applications or ingestion of this substance may cause neurotoxic effects.

There are two systemic agents for treating pediculosis capitis. Trimethoprim-Sulfamethoxazole (80mg trimethoprim/400mg sulfamethoxazole) and 200mcg/kg in a single dose. These systemic agents may be used when there is little or no response to other treatment, and when problems arise in applying topical treatment.

In brief, it may be said that today pediculosis treatment resorts to organochlorine products, organophosphorated products and permethrins, all of them having a high degree of toxicity which prevents the use thereof in pregnant women and calls for special care when applied on children. Moreover, it has been demonstrated that these agents generate a high degree of resistance in lice, reducing treatment effectiveness. This increased resistance requires treatment repetition in short time periods. Sanitary authorities worldwide have recognized that this poses a serious medical and social problem, since pediculosis prevalence in humans is very high. It is assumed that pediculosis prevalence in Argentina ranges between 40 to 80% according to the different regions. In 2001 it was estimated that there were between 6 and 12 million infested persons in U.S.A.

Therefore, there is a need for pediculosis treatment that is non toxic for human beings and that fails to generate lice resistance.

Garlic is a herbaceous species, *Allium satívum L.* (Liliaceae), from central Asia. It has been used since ancient times in culinary and pharmacological applications as an antiseptic, hypotensive, diuretic, etc., agent.

Garlic contains mineral salts, sugars, lipids, proteins, saponosides, terpenes, vitamins, enzimes, and sulphur compounds as active principles. If the bulb is fresh and intact, the majority compound is alliine or S-allil-cysteine sulphoxide (a sulphureous amino acid). When garlic is crushed or cut, alliine is transformed in allicin by the action of the allinase enzime or S-alkyl-cisteyne sulphoxideliase. Allicine oxidates and produces diallil disulphur, a main component of its characteristic aroma. Garlic also contains ajoenic compounds, formed by the condensation of allicin.

Traditionally, garlic has been used as an antiseptic, antihelminthic, expectorant, diuretic, antibacterian, etc. agent. In the last years, garlic's beneficial properties over cholesterolemia and triglyceridemia and its antihypertensive effects have been demonstrated. It is believed that the latter property is due to a reduction in factors contributing to blood pressure control, for example, vasoconstricting agents - prostaglandines and antiotensine II.

It has also been demonstrated that garlic has inhibitory properties over *in vitro* platelet aggregation. Some investigators attribute this property to ajoenic compounds acting as lipoxigeriase inhibitors.

Although the results of the different clinical tests are not final, maybe because of the differences between the preparations used or the protocols followed in the research, it is considered that standardized garlic preparations have a mild hypotensive effect on hypertensive persons, fibrinolytic activity, reduce seric lipids and, thus, the atherosclerosis process.

Also, results on the clinical effects of garlic as an antithrombotic, vasodilatation and anticarcinogenic agent have been published.

Acute and chronic toxicity tests in mice and rats have demonstrated that garlic extracts are atoxic. In human beings it is regarded as an atoxic drug; however, it may cause digestion disorders in some persons.

Furthermore, allicin extracted from garlic is used as a fungicide inhibiting growth of at least 10 pathogen fungi species; as a bactericidal and antiparasitary agent, specially against nemathods. The effectiveness against aphides, particularly against «plant louse» has also been demonstrated and, if administered orally, the number of tick bites decreases.

As far as inventors know, currently there is no pediculosis treatment based on garlic extracts, specially on allicin and other sulphur compounds extracted from garlic.

### BRIEF DESCRIPTION OF THE INVENTION

Thus it is an object of the present invention to provide a topical application composition for preventing and treating pediculosis, which comprises at least between 0.2 and 0.3% (w/v) of diallil tiosulphinate (allicin), wherein the diallil tiosulphinate compound is extracted from the garlic plant (*Allium sativum sp*). The composition may also comprise between 0.06 and 0.1 % (w/v) of other tiosulphinates and sulphur compounds derived from methyl methane tiosulphinate, allil methane tiosulphinate, methyl 2-propene tiosulphinate, trans 1-propenyl methane tiosulphinate, methyl trans 1 propene tiosulphinate, allil 2-propene tiosulphinate, allil trans-1propene tiosulphinate, trans-1 propenyl 2 propene tiosulphinate or combinations thereof, all of them extracted from the garlic plant (*Allium sativum sp*).

According to the formulation desired, the composition of the invention may also comprise additives such as emulsifying agents, aromatic agents, binders, colorants, preserving agents, bactericidal and fungicidal agents, surfactants, greasing agents and viscosity agents. Such formulations may be in form of hair lotions, hair creams, hair conditioners, shampoo, hair rinse lotions, hair gel or powder for topical application and in any other cosmetic form included in Resolution 155/98, National Ministry of Public Health.

In another object of the present invention a method is provided for preparing the composition of the invention, said method comprising the steps of:
a) Peeling and grinding garlic cloves until obtaining a homogeneous paste;
b) Heating an amount of water up to boiling point and adding an amount of an organic acid, e.g. citric acid, until making up an aqueous solution with a pH ranging between 3 and 3.5;
c) Immersing an amount of the garlic paste obtained in stage a) into an amount of the boiling aqueous solution of stage b) ; and
d) Cooling and fractioning the aqueous solution in stage c), wherein said aqueous solution comprises diallil tiosulphinate and other tiosulphinate compounds and sulphur compounds deriving therefrom.

In another object of the present invention, the use of the composition of the invention for elaborating a medicine or a cosmetic product against pediculosis is provided, wherein said medicine or cosmetic product is presented as a hair lotion, a hair cream, a hair conditioner, a shampoo, a hair rinse lotion, a hair gel and a topical application powder and any other cosmetic form contemplated in Resolution 155/98, National Ministry of Public Health.

### DESCRIPTION OF THE DRAWINGS

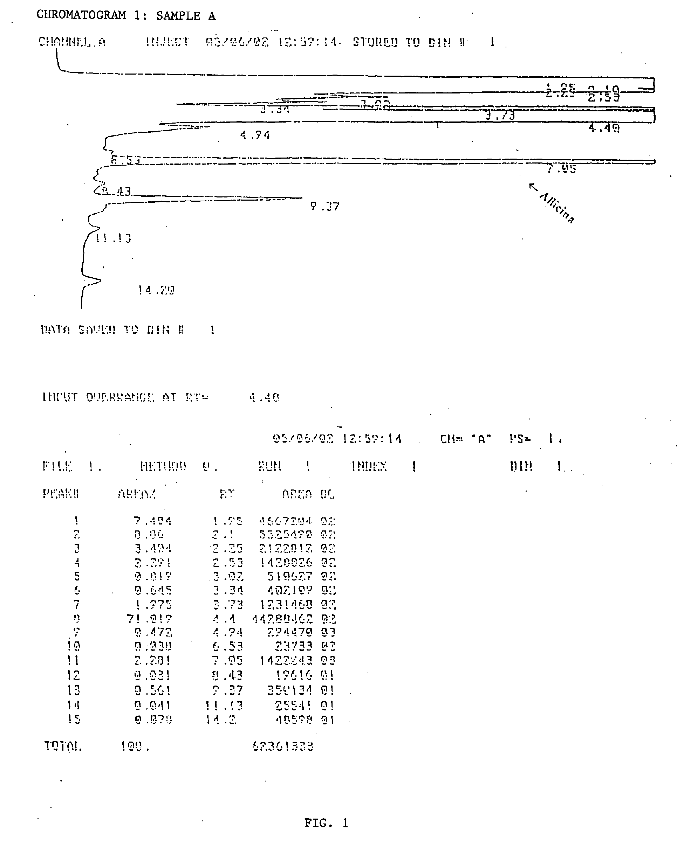

Figure 1: shows Sample A chromatogram and the allicin peak is indicated. Sample A was obtained pursuant to the method described in Example 1.

### DETAILED DESCRIPTION OF THE INVENTION

The composition of the present invention acts as a repellent, a pediculiside and a nit remover of the *Pediculus humanus capitis* and *Phtirus pubis* varieties. The composition is atoxic, has no contraindications and does not generate resistance. It can be applied to children and pregnant women on a regular basis. Elaboration can be made at a low cost and it constitutes a powerful tool for preventing and treating pediculosis.

When the composition of the invention was applied on infested children an average 96% reduction of the lice population was recorded 7 days after treatment had started and with a single application. No side effects such as skin irritation, eye irritation, allergy or product intolerance were evident.

It must be pointed out that lice removal was achieved even in high infestation cases, for example, two children having 122 and 152 lice, respectively, at the beginning of the treatment.

When used at low allicin concentrations, the composition of the invention acts as a lice repellant. On the other hand, when higher concentrations of the active principle (allicin) are used, the composition acts as a pediculiside.

The HPLC analysis of the composition of the invention obtained pursuant to Example 1 herein, showed in two separate analysis that said composition contains between 2.02 and 2.60 mg/ml allicin.

For the purposes of this application, the terms "allicin" and "diallil tiosulphinate" shall be considered equivalent, and, therefore, interchangeable.

In a preferred embodiment, the composition of the invention was prepared as a hair conditioner formulation (Table 1).

**Table 1**

| **Components** | **Components pursuant to INCI** | **%** |
|---|---|---|
| Water | Aqua | 80.70 |
| Vaseline | Petrolatum | 10.00 |
| Cethyl trimethyl ammonium 25 | Cetromonium chloride 25% | 4.00 |
| Cetyl alcohol | Cetyl alcohol | 3.00 |
| Garlic (allicin) | Allium Sativum (Garlic) extract | 1.00 |
| Citric acid | Citric acid | 1.00 |
| Nipagin | Methylparaben | 0.20 |
| Aroma | Parfum | 0.10 |

The hair conditioner of the invention has the following features: a 3.5 pH, a density equal to 0.98 g/m ± 0.02 at 20°C, and a viscosity equal to ± 200 centipoise at 20°C.

Any quaternary ammonium compound can be used as an emulsifying agent without altering the spirit of this invention. Oil agents other than cetyl alcohol disclosed in Table 1 can be used, for example stearyl alcohol, cetyl stearyl alcohol, petrolatum, or other that may adequately fulfill the hair aspect improvement purpose.

The composition of the invention can also be used to elaborate a hair cream. The formulation of the hair cream is similar to the formulation of hair conditioner, but with a pH not lower than 5.5.

The composition of the invention can also be used to elaborate a shampoo. In a preferred embodiment, the shampoo of the invention comprises a tensioactive or surfactant agent such as sodium lauryl ether sulfate or triethanolamine lauryl sulfate; a greasing agent such as coconut diethanolamide; additives such as silicones, propylene glycol, esters, proteins and mixtures thereof; a viscosity agent such as sodium chloride or methyl cellulose; the composition of the invention that comprises, among other sulfur compounds, allicin; aromatic agents, such as mint and preserving agents, such as formol or nipagin, where the shampoo pH is about 6.

The composition of the invention can also be used to elaborate a hydroalcoholic hair lotion consisting of water, 90° alcohol, the composition of the invention, comprising, among other sulfur compounds, allicin and an aromatic agent such as mint.

This invention is better illustrated in the following examples, which should not be viewed as narrowing the scope hereof. On the contrary, it should be clearly understood that other embodiments, modifications and equivalent applications can be made after reading the present description, that may be suggested to those skilled in the art without departing from the spirit of the present invention and/or the scope of the attached claims.

### Examples

### Example 1: Elaboration of the composition of the invention by extraction of allicin and sulfur compounds from garlic

Garlic cloves are peeled and ground until a homogeneous paste is obtained.

In turn, water is heated until boiling point and citric acid is added until the solution pH reaches a 3.5 value.

The garlic paste is introduced in the boiling aqueous solution of the previous stage and it is maintained during 30 seconds. The quantity of garlic paste and aqueous solution are in equal parts.

The garlic paste is then removed and the aqueous solution is cooled and separated in aliquots until use.

### Example 2: Cromatographic analysis of the composition of the invention

Two different samples of the composition of the invention obtained pursuant to the method described in Example 1, were analyzed by means of HPLC.

The samples were kept in the refrigerator until use. The analysis was made in a High Precision Liquid Chromatograph Konik A400, UV 254nm, Column Waters RPC18 and mobile phase methanol-water 50:50. The flow was equal to 1 ml/min.

### Example 3 Clinical tests using the composition of the invention

Clinical tests were carried out in 90 children of age ranging between 2 and 4 years old, both sexes. Two survey groups were formed, one with a high degree of infestation and another one with a low or medium degree of infestation. For each group a control group of children with no lice was constituted.

The composition of the invention, as obtained in Example 1, was applied on the scalp of children in both groups. The composition was allowed to act during 10 minutes. Then hair was washed and a thin comb was passed to each child. Treatment was applied again 7 days later. The control group received no treatment.

In order to evaluate the number of lice the following method was used: a cap covering the head and a paper sheet around the neck and shoulders were placed. Lice stuck to the cap and those falling from the head on to the paper sheet were counted. The same method was applied to control groups.

Lice accounting was taken after application of the composition of the invention and 7 days later, when the treatment was completed.

### Exemple 4: Use of the composition of the invention for the elaboration of a hair conditioner or hair rinse cream

Mix 1: 5% Glyceril monostearate together with a mix of the following aqueous components: 90% water containing allicin - tiosulphinates and sulfur compounds deriving therefrom up to a proportion comprising between 0.2 and 0.3% (w/v) expressed in gr/100 ml, and other tiosulphinates between 0.06 - 0.1% (w/v) expressed in gr/100 ml, is heated up to a temperature ranging between 70° and 75°C.

Mix 2: On a separate basis, 3% propylene glycol, with the addition of 2% Nipagin (a preserving agent) is heated up to a temperature ranging between 70° and 75°C.

Mixes 1 and 2 are then combined at a temperature of 50°C, they are cooled and then citric acid is added until a 3.5 pH is reached.

### Example 5: Use of the composition of the invention for the elaboration of a hair cream.

The hair cream is prepared on a similar basis as Example 4, then citric acid is added until a pH not lower than 5.5 is reached.

### Example 6: Use of the composition of the invention for the elaboration of a shampoo

Up to 68% cold water, containing allicin - tiosulphinates and sulfur compounds deriving therefrom up to a proportion comprising between 0.2 and 0.3% (w/v) expressed in g/100 ml, and other tiosulphinates between 0.06 and 0.1% (w/v) expressed in g/100 ml, are mixed. Triethanolamine lauryl sulfate up to 30%, with 6% coconut diethanolamide, 2% nipagin and parfum (mint or other aromatic agents), as required, are added. Once mixed, citric acid is added until pH 6 is reached.

### Example 7: use of the composition of the invention for the elaboration of a hair lotion

The following components are mixed, cold: up to 64% water containing allicin - tiosulphonates between 0.2 and 0.3% (w/v), expressed in g/100 ml, and other tiosulphinates between 0.06% and 0.1% (w/v), expressed in g/100 ml, and other tiosulphinates between 0.06% and 0.1% (w/v), expressed in g/100 ml, with 25% 90° alcohol, 5% glycerin, 4% castor oil and 2% mint aromatic agent (or parfum). Once the components have been mixed, citric acid is added until pH 3.5 is reached.

## Claims

1. A topical application composition for the prevention and treatment of pediculosis, comprising between 0.2 and 0.3% (w/v) of diallil tiosulphinate, wherein the tiosulphinate diallil compound is extracted from the garlic plant (*Allium sativum* sp).

2. The composition according to claim 1 comprising selected additives from the group consisting of emulsifying agents, aromatic agents, bonding agents, colorants, preserving agents, bactericidal agents and fungicidal agents, surfactants, greasing agents, viscosity agents and combinations thereof.

3. The composition according to claim 2 wherein it is used to elaborate a product from the group comprising hair lotions, hair creams, hair conditioners, shampoo, hair rinse cream, hair gel and topical application powder.

4. The use of the composition of claim 3, characterized because said composition is used for the elaboration of a medicine for prevention and treatment of pediculosis.

5. The use of the composition of claim 3, characterized because said composition is used for the elaboration of a cosmetic product for prevention of pediculosis.

6. The composition according to claim 1, comprising also between 0.06% and 0.1% (w/v) of other tiosulphinates and sulfur compounds deriving therefrom, all of them extracted from the garlic plant (*Allium sativum* sp).

7. The composition according to claim 6 comprising selected additives from the group consisting of emulsifying agents, aromatic agents, bonding agents, colorants, preserving agents, bactericidal agents and fungicidal agents, surfactants, greasing agents, viscosity agents and combinations thereof.

8. The composition according to claim 7 where it is used to elaborate a product from the group comprising hair lotions, hair creams, hair conditioners, shampoo, hair rinse cream, hair gel and topical application powder.

9. The use of the composition of claim 8, characterized because said composition is used for the elaboration of a medicine for prevention and treatment of pediculosis.

10. The use of the composition of claim 8, characterized because said composition is used for the elaboration of a cosmetic product for prevention of pediculosis.

11. A method for preparation of a diallil tiosuphinate, and other tiosulphinates and sulfer compounds comprises the stages of:
a) peeling and grinding garlic cloves until a homogeneous paste is obtained;
b) heating a quantity of water to its boiling point, adding a quantity of organic acid until obtaining an aqueous solution with pH between 3 and 3.5;
c) immersing a quantity of the garlic paste obtained in stage a) in a quantity of the boiling aqueous solution of stage b); and
d) cooling and fractioning the aqueous solution of stage c), wherein said aqueous solution comprises diallil tiosulphinate and other tiosuphinate and sulfur compounds

12. The method according to claim 11, wherein the garlic paste of stage a) and the aqueous solution of stage b) are in equal parts.

13. The method according to claim 11, wherein the aqueous solution of stage c) contains between 1mg/ml and 4 mg/ml of diallil tiosulphinate.

14. The method according to claim 11, wherein the organic acid is selected from the group comprised by citric acid, ascorbic acid and combinations thereof.

15. The method according to claim 14, wherein the organic acid is citric acid.
